(19) Europäisches Patentamt
European Patent Office
Office européen des brevets

(11) Veröffentlichungsnummer: **0 164 633**
**A2**

(12) # EUROPÄISCHE PATENTANMELDUNG

(21) Anmeldenummer: **85106491.5**

(22) Anmeldetag: **25.05.85**

(51) Int. Cl.[4]: **C 07 D 401/06**
A 61 K 31/40

(30) Priorität: **09.06.84 DE 3421641**

(43) Veröffentlichungstag der Anmeldung:
**18.12.85 Patentblatt 85/51**

(84) Benannte Vertragsstaaten:
**AT BE CH DE FR GB IT LI NL SE**

(71) Anmelder: **Merck Patent Gesellschaft mit beschränkter Haftung**
**Frankfurter Strasse 250**
**D-6100 Darmstadt(DE)**

(72) Erfinder: **Hausberg, Hans-Heinrich, Dr.**
**Odenwaldstrasse 30**
**D-6105 Ober-Ramstadt(DE)**

(72) Erfinder: **Böttcher, Henning, Dr.**
**Soderstrasse 95**
**D-6100 Darmstadt(DE)**

(72) Erfinder: **Seyfried, Christoph, Dr.**
**Mathildenstrasse 6**
**D-6104 Seeheim-Jugenheim(DE)**

(72) Erfinder: **Minck, Klaus-Otto, Dr.**
**Büchestrasse 8**
**D-6105 Ober-Ramstadt(DE)**

(54) **Indolderivate.**

(57) Neue Indolderivate der Formel I

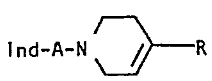

I

worin

Ind einen unsubstituierten oder ein- bis dreifach durch Alkyl, O-Alkyl, S-Alkyl, OH, F, Cl und/oder Br substituierten Indol-4-yl-rest,

A $-C_nH_{2n}-$ oder $-CHCH-CH_2-$,

R einen unsubstituierten oder ein- oder sweifach durch Alkyl, F, Cl, Br und/oder $CF_3$ substituierten Phenyl- oder 2- oder 3-Thienylrest und

n 2,3 oder 4

bedeuten und die Alkylgruppen jeweils 1 - 4 C-Atome besitzen,

sowie deren Salze zeigen dämpfende Wirkungen auf das Zentralnervensystem, insbesondere neuroleptische Wirkungen.

EP 0 164 633 A2

Croydon Printing Company Ltd

# Indolderivate

Die Erfindung betrifft neue Indolderivate der Formel I

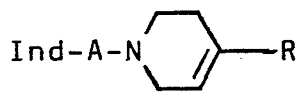

I

worin

Ind einen unsubstituierten oder ein- bis dreifach durch Alkyl, O-Alkyl, S-Alkyl, OH, F, Cl und/oder Br substituierten Indol-4-yl-rest,

A $-C_nH_{2n}-$ oder $-CH{=}CH-CH_2-$,

R einen unsubstituierten oder ein- oder zweifach durch Alkyl, F, Cl, Br und/oder $CF_3$ substituierten Phenyl- oder 2- oder 3-Thienylrest und

n 2,3 oder 4

bedeuten und die Alkylgruppen jeweils 1 - 4 C-Atome besitzen,
sowie deren Salze.

Der Erfindung lag die Aufgabe zugrunde, neue Verbindungen aufzufinden, die zur Herstellung von Arzneimitteln verwendet werden können.

Es wurde gefunden, daß die genannten Substanzen bei guter Verträglichkeit wertvolle pharmakologische Eigenschaften besitzen. So zeigen sie z.B. das Zentralnervensystem beeinflussende, vorzugsweise dämpfende (z.B. sedierende, tranquillierende, neuroleptische und/oder antidepressive)Wirkungen. Im einzelnen haben die Verbindungen eine dämpfende Wirkung auf das Verhalten bei Mäusen (Methodik vgl. Irwin, Psychopharmacologia 13 (1968), 222-257), hemmen bei Mäusen das durch

Apomorphin induzierte Kletterverhalten (Methodik vgl. Costall et al., European J. Pharmacol. 50 (1968), 39-50) und induzieren contralaterales Drehverhalten in Hemiparkinson-Ratten (feststellbar nach der Methode von Ungerstedt et al., Brain Res. 24 (1970), 485-493), ohne daß nennenswerte kataleptische Nebenwirkungen auftreten (Methodik vgl. Dolini-Stola, Pharmakopsychiat. 6 (1973), 189-197). Weiterhin hemmen die Substanzen die Bindung von tritiierten Dopaminagonisten und -antagonisten an striäre Rezeptoren (feststellbar nach der Methode von Schwarcz et al., J. Neurochemistry 34 (1980), 772-778,und Creese et al., European J. Pharmacol. 46 (1977), 377-381). Zusätzlich hemmen die Verbindungen den Zungen-Kieferreflex bei der narkotisierten Ratte (feststellbar in Anlehnung an die Methoden von Barnett et al., European J. Pharmacol. 21 (1973), 178-182, und von Ilhan et al., European J. Pharmacol. 33 (1975), 61-64). Weiterhin treten analgetische und blutdrucksenkende Wirkungen auf; so wird der bei katheter-tragenden wachen, spontan hypertonen Ratten (Stamm SHR/NIH-MO//CHB-EMD; Methode vgl. Weeks und Jones, Proc. Soc. Exptl. Biol. Med. 104 (1960), 646-648) direkt gemessene arterielle Blutdruck nach intragastraler Gabe der Verbindungen gesenkt.

Verbindungen der Formel I und ihre physiologisch unbedenklichen Säureadditionssalze können daher als Arzneimittelwirkstoffe und auch als Zwischenprodukte zur Herstellung anderer Arzneimittelwirkstoffe verwendet werden.

Gegenstand der Erfindung sind die Indolderivate der Formel I sowie ihre Salze.

In den Resten Ind und R bedeutet Alkyl vorzugsweise Methyl, ferner auch Ethyl, n-Propyl, Isopropyl, n-Butyl, Isobutyl, sek.-Butyl oder tert.-Butyl. O-Alkyl ist vorzugsweise Methoxy, ferner auch Ethoxy, n-Propoxy, Isopropoxy, n-Butoxy, Isobutoxy, sek.-Butoxy oder tert.-Butoxy. S-Alkyl ist vorzugsweise Methylthio, ferner auch Ethylthio, n-Propylthio, Isopropylthio, n-Butylthio, Isobutylthio, sek.-Butylthio oder tert.-Butylthio.

Der Rest Ind bedeutet insbesondere einen unsubstituierten, ferner einen einfach substituierten Indol-4-yl-rest.Vorzugsweise ist er in der 2- oder 5-Stellung oder auch in der 1-, 3-, 6- oder 7-Stellung substituiert. Bevorzugte disubstituierte Indol-4-yl-reste sind in 2,5-Stellung substituiert; Disubstitution ist auch in 1,2-, 1,3-, 1,5-, 1,6-, 1,7-, 2,3-, 2,6-, 2,7-, 3,5-, 3,6-, 3,7-, 5,6-, 5,7- oder 6,7-Stellung möglich, Trisubstitution in 1,2,3-, 1,2,5-, 1,2,6-, 1,2,7-, 1,3,5-, 1,3,6-, 1,3,7-, 1,5,6-, 1,6,7-, 2,3,5-, 2,3,6-, 2,3,7-, 2,5,6-, 2,6,7-, 3,5,6-, 3,6,7- oder 5,6,7-Stellung. In allen diesen Fällen können die Substituenten gleich oder verschieden sein.

Im einzelnen sind die bevorzugten Substituenten im Rest Ind Methyl, Ethyl, Methoxy, Ethoxy, Methylthio, Ethylthio, OH, F, Cl und/oder Br. Bevorzugte Bedeutungen des Restes Ind sind 5-Methoxyindol-4-yl, 3-Methylindol-4-yl und 2-Hydroxyindol-4-yl(= Oxindol-4-yl), ferner 1-, 2-, 5-, 6- und 7-Methyl-indol-4-yl, 1-, 2-, 4-, 5-, 6- und 7-Ethylindol-4-yl, 2-, 3-, 6- und 7-Methoxy-indol-4-yl, 2-, 3-, 5-, 6- und 7-Ethoxy-indol-4-yl, 5-, 6- und 7-Methylthioindol-4-yl, 5-, 6- und 7-Ethylthio-indol-4-yl, 3-, 5-, 6- und 7-Hydroxy-indol-4-yl, 5-, 6- und 7-Fluor-

indol-4-yl, 5-, 6- und 7-Chlorindol-4-yl, 5-, 6- und 7-Bromindol-4-yl, 1,2-, 1,3-, 1,5-, 1,6-, 1,7- 2,3-, 2,5-, 2,6-, 2,7-, 3,5-, 3,6-, 3,7-, 5,6-, 5,7- und 6,7-Dimethylindol-4-yl, 1-Methyl-2-, -3-, -5-, -6- und -7-ethylindol-4-yl, 1-Methyl-5-, -6- und -7-methoxyindol-4-yl, 1-Methyl-5-, -6- und -7-ethoxyindol-4-yl, 1-Methyl-5-, -6- und -7-methylthioindol-4-yl, 1-Methyl-5-, -6- und -7-ethylthioindol-4-yl, 1-Methyl-2-, -3-, -5-, -6- und -7-hydroxyindol-4-yl, 1-Methyl-5-, -6- und -7-fluorindol-4-yl, 1-Methyl-5-, -6- und -7-chlorindol-4-yl, 1-Methyl-5-, -6- und -7-bromindol-4-yl, 1-Ethyl-2-methylindol-4-yl, 2-Methyl-3-, -5-, -6- und -7-ethylindol-4-yl, 2-Methyl-5-, -6- und -7-methoxyindol-4-yl, 2-Methyl-5-, -6- und -7-ethoxyindol-4-yl, 2-Methyl-5-, -6- und -7-methylthioindol-4-yl, 2-Methyl-5-, -6- und -7-ethylthioindol-4-yl, 2-Methyl-5-, -6- und -7-hydroxyindol-4-yl, 2-Methyl-5-, -6- und -7-fluorindol-4-yl, 2-Methyl-5-, -6- und -7-chlorindol-4-yl, 2-Methyl-5-, -6- und -7-bromindol-4-yl. 3-Methyl-5-, -6- und -7-methoxyindol-4-yl, 5,6-, 5,7- und 6,7-Dimethoxyindol-4-yl, 5-Methoxy-6- und -7-fluorindol-4-yl, 5-Methoxy-6- und -7-chlorindol-4-yl, 5-Methoxy-6- und -7-bromindol-4-yl, 2-Hydroxy-3-, -5-, -6- und -7-methylindol-4-yl, 2-Hydroxy-3-, -5-, -6- und -7-methoxyindol-4-yl. Der Parameter n ist vorzugsweise 4, der Rest A ist vorzugsweise $-CH=CH-CH_2-$ (insbesondere in trans-Konfiguration), weiterhin vorzugsweise $-(CH_2)_2-$, $-(CH_2)_3-$ oder $-(CH_2)_4$.

Der Rest R ist bevorzugt unsubstituiertes Phenyl. Falls R eine substituierte Phenylgruppe bedeutet, so ist diese vorzugsweise einfach substituiert.

Sie kann jedoch auch zweifach substituiert sein, wobei die Substituenten gleich oder verschieden sein können. Bevorzugte Substituenten an der Phenylgruppe sind Methyl und Cl. Im einzelnen ist R bevorzugt Phenyl, o-, m- oder p-Tolyl, o-, m- oder p-Ethylphenyl, o-, m- oder p-Fluorphenyl, o-, m- oder p-Chlorphenyl, o-, m- oder p-Bromphenyl, o-, m- oder p-Trifluormethylphenyl, 2,3-, 2,4-, 2,5-, 2,6-, 3,4- oder 3,5-Dimethylphenyl. 2,3-, 2,4-, 2,5-, 2,6-, 3,4- oder 3,5-Dichlorphenyl, 2-Methyl-4-chlorphenyl, 2- oder 3-Thienyl, 5-Methyl-2-thienyl.

Dementsprechend sind Gegenstand der Erfindung insbesondere diejenigen Verbindungen der Formel I, in denen mindestens einer der genannten Reste eine der vorstehend angegebenen, insbesondere der vorstehend angegebenen bevorzugten Bedeutungen hat. Einige bevorzugte Gruppen von Verbindungen können durch die folgenden Teilformeln Ia bis If ausgedrückt werden, die der Formel I entsprechen und worin die nicht näher bezeichneten Reste und Parameter die bei Formel I angegebene Bedeutung haben, worin jedoch

| | | |
|---|---|---|
| in Ia | Ind | Indol-4-yl, Methylindol-4-yl, Hydroxyindol-4-yl oder Methoxyindol-4-yl bedeutet, wobei die Substituenten vorzugsweise in 2-, 3- oder 5-Stellung stehen; |
| in Ib | Ind | Indol-4-yl, 3-Methylindol-4-yl, 2-Hydroxyindol-4-yl oder 5-Methoxyindol-4-yl bedeutet |
| in Ic | A | $-(CH_2)_n-$ oder $-CH=CH-CH_2-$ bedeutet; |
| in Id | R | Phenyl bedeutet; |
| in Ie | Ind | Indol-4-yl, 3-Methylindol-4-yl, 2-Hydroxyindol-4-yl oder 5-Methoxyindol-4-yl, |

A $-(CH_2)_n-$ oder $-CH=CH-CH_2-$ und

R Phenyl

bedeuten;

in If Ind Indol-4-yl,

A $-(CH_2)_n-$ oder $-CH=CH-CH_2-$ und

R Phenyl

bedeuten.

Einige Verbindungen der Formel I können ein oder mehrere asymmetrische Kohlenstoffatome besitzen. Sie können daher als Racemate, falls mehrere asymmetrische Kohlenstoffatome vorhanden sind, auch als Gemische mehrerer Racemate sowie in verschiedenen optisch-aktiven Formen vorliegen.

Gegenstand der Erfindung ist ferner das in Patentanspruch 3 beschriebene Verfahren zur Herstellung der Verbindungen der Formel I sowie ihrer Salze.

Die Herstellung der Verbindungen der Formel I erfolgt im übrigen nach an sich bekannten Methoden, wie sie in der Literatur (z. B. in den Standardwerken wie Houben-Weyl, Methoden der Organischen Chemie, Georg-Thieme-Verlag, Stuttgart; Organic Reactions, John Wiley & Sons, Inc., New York) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe für das beanspruchte Verfahren können gewünschtenfalls auch in situ gebildet werden, derart, daß man sie aus dem Reaktionsgemisch nicht isoliert, sondern sofort weiter zu den Verbindungen der Formel I umsetzt.

In den Indolderivaten der Formel II ist $X^1$ vorzugsweise X; dementsprechend sind in den Verbindungen der Formel III $X^2$ und $X^3$ vorzugsweise zusammen NH. Der Rest X ist vorzugsweise Cl oder Br; er kann jedoch auch J, OH oder eine reaktionsfähig funktionell abgewandelte OH-Gruppe bedeuten, insbesondere Alkylsulfonyloxy mit 1-6 (z. B. Methansulfonyloxy) oder Arylsulfonyloxy mit 6-10 C-Atomen (z. B. Benzolsulfonyloxy, p-Toluolsulfonyloxy, 1- oder 2-Naphthalin-sulfonyloxy).

Dementsprechend sind die Indolderivate der Formel I insbesondere durch Umsetzung von Verbindungen der Formel Ind-A-Cl oder Ind-A-Br mit Tetrahydropyridinderivaten der Formel III, worin $X^2$ und $X^3$ zusammen eine NH-Gruppe bedeuten (nachstehend als IIIa bezeichnet) erhältlich.

Die Verbindungen der Formeln II und insbesondere III sind zum Teil bekannt; die nicht bekannten Verbindungen der Formeln II und III können leicht analog zu den bekannten Verbindungen hergestellt werden. Primäre Alkohole der Formel Ind-A-OH sind z.B. durch Reduktion der entsprechenden Carbonsäuren oder ihrer Ester erhältlich. Behandeln mit Thionylchlorid, Bromwasserstoff, Phosphortribromid oder ähnlichen Halogenverbindungen liefert die entsprechenden Halogenide der Formel Ind-A-Hal. Die entsprechenden Sulfonyloxyverbindungen sind erhältlich aus den Alkoholen Ind-A-OH durch Umsetzung mit den entsprechenden Sulfonsäurechloriden. Die Jodverbindungen der Formel Ind-A-J sind z.B. durch Einwirkung von Kaliumjodid auf die zugehörigen p-Toluolsulfonsäureester erhältlich. Die Amine der Formel Ind-A-$NH_2$ sind z.B. aus den Halogeniden mit Phthalimidkalium oder durch Reduktion der entsprechenden Nitrile herstellbar.

Die Tetrahydropyridinderivate IIIa sind größtenteils bekannt (vgl. DE-OS 20 60 816) und z.B. erhältlich durch Umsetzung von 4-Piperidon mit metallorganischen Verbindungen der Formel M-R (worin M ein Li-Atom oder MgHal bedeutet), anschließende Hydrolyse zu den entsprechenden 4-R-4-hydroxypiperidinen sowie nachfolgende Dehydratisierung zu 4-R-3,4-dehydro-piperidinen. Verbindungen der Formel III ($X^2$ und $X^3$ = jeweils X) sind z.B. herstellbar durch Reduktion von Diestern der Formel $AlkylOOC-CH_2-CR=CH-COOAlkyl$ zu Diolen der Formel $HO-CH_2CH_2-CR=CH-CH_2OH$ (III, $X^1 = X^2 = OH$) und gegebenenfalls anschließende Umsetzung mit $SOCl_2$ bzw. $PBr_3$.

Die Umsetzung der Verbindungen II und III verläuft nach Methoden, wie sie für die Alkylierung von Aminen aus der Literatur bekannt sind. Man kann ohne Gegenwart eines Lösungsmittels die Komponenten miteinander verschmelzen, gegebenenfalls im geschlossenen Rohr oder im Autoklaven. Es ist aber auch möglich, die Verbindungen in Gegenwart eines indifferenten Lösungsmittels umzusetzen. Als Lösungsmittel eignen sich z. B. Kohlenwasserstoffe, wie Benzol, Toluol, Xylol; Ketone wie Aceton, Butanon; Alkohole wie Methanol, Ethanol, Isopropanol, n-Butanol; Ether wie Tetrahydrofuran (THF) oder Dioxan; Amide wie Dimethylformamid (DMF) oder N-Methyl-pyrrolidon; Nitrile wie Acetoniril, gegebenenfalls auch Gemische dieser Lösungsmittel untereinander oder Gemische mit Wasser. Der Zusatz eines säurebindenden Mittels, beispielsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums oder Calciums, oder

der Zusatz einer organischen Base wie Triethylamin, Dimethylanilin, Pyridin oder Chinolin oder eines Überschusses der Aminkomponente Ind-A-$NH_2$ bzw. des Piperidinderivates der Formel IIIa kann günstig sein. Die Reaktionszeit liegt je nach den angewendeten Bedingungen zwischen einigen Minuten und 14 Tagen, die Reaktionstemperatur zwischen etwa 0 und 150°, normalerweise zwischen 20 und 130°.

Es ist ferner möglich, eine Verbindung der Formel I zu erhalten, indem man ein Vorprodukt, das an Stelle von Wasserstoffatomen eine oder mehrere reduzierbare Gruppe(n) und/oder eine oder mehrere zusätzliche C-C- und/oder C-N-Bindung(en) enthält, mit einem reduzierenden Mittel behandelt, vorzugsweise bei Temperaturen zwischen -80 und +250° in Gegenwart mindestens eines inerten Lösungsmittels.

Reduzierbare (durch Wasserstoff ersetzbare) Gruppen sind insbesondere Sauerstoff in einer Carbonylgruppe, Hydroxyl, Arylsulfonyloxy (z. B. p-Toluolsulfonyloxy), N-Benzolsulfonyl, N-Benzyl oder O-Benzyl.

Es ist grundsätzlich möglich, Verbindungen, die nur eine, oder solche, die nebeneinander zwei oder mehr dieser Gruppen bzw. zusätzlichen Bindungen enthalten, reduktiv in eine Verbindung der Formel I überzuführen. Vorzugsweise bedient man sich hierzu des nascierenden Wasserstoffs oder komplexer Metallhydride, ferner der Reduktion nach Wolff-Kishner.

Bevorzugte Ausgangsstoffe für die Reduktion entsprechen der Formel

Ind'-L-Q V

worin

Ind' - einen unsubstituierten oder ein- bis dreifach durch Alkyl, O-Alkyl, S-Alkyl, OH, F, Cl, Br und/oder O-Benzyl und/oder durch eine Arylsulfonylgruppe oder eine Benzylgruppe in 1-Stellung substituierten Indol-4-ylrest,

L A oder eine dem Rest A entsprechende Kette, worin jedoch eine oder mehrere $-CH_2$-Gruppe(n) durch -CO- und/oder ein oder mehrere Wasserstoffatome durch OH-Gruppen ersetzt sind,

Q 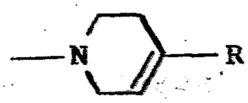 oder 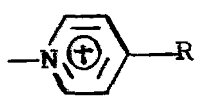 $An^{\ominus}$ und

$An^{\ominus}$ ein Anion einer starken Säure bedeuten,

worin jedoch nicht gleichzeitig Ind' = Ind, L = A und

Q = 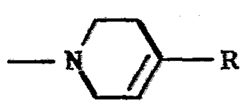 sein können.

In den Verbindungen der Formel V ist L bevorzugt $-(CH_2)_{n-1}-CO-$ [im einzelnen $-CH_2CO-$, $-CH_2CH_2-CO-$ oder $-(CH_2)_3-CO-$] oder -CH=CH-CO-, ferner z.B. $-CO-(CH_2)_{n-2}-CO-$ [im einzelnen -COCO-, $-COCH_2CO-$ oder $-CO-(CH_2)_2-CO-$], $-CO-CH_2CH_2-$, $-CH_2CO-CH_2-$, $-CO-(CH_2)_3-$, $-CH_2-CO-CH_2CH_2-$ oder $-CH_2CH_2-CO-CH_2-$.

Verbindungen der Formel V sind z.B. herstellbar durch Umsetzung von IIIa mit einer Verbindung der Formel VI

$$Ind'-L-X^1 \qquad VI$$

worin

Ind', L und $X^1$ die oben angegebenen Bedeutungen haben,

unter den Bedingungen, die oben für die Umsetzung von II und III angegeben sind.

Wird als Reduktionsmittel nascierender Wasserstoff verwendet, so kann man diesen z. B. durch Behandlung von Metallen mit schwachen Säuren oder mit Basen erzeugen. So kann man z. B. ein Gemisch von Zink mit Alkalilauge oder von Eisen mit Essigsäure verwenden. Geeignet ist auch die Verwendung von Natrium oder einem anderen Alkalimetall in einem Alkohol wie Ethanol, Isopropanol, Butanol, Amyl- oder Isoamylalkohol oder Phenol. Man kann ferner eine Aluminium-Nickel-Legierung in alkalisch-wässeriger Lösung, gegebenenfalls unter Zusatz von Ethanol, verwenden. Auch Natrium- oder Aluminiumamalgam in wässerig-alkoholischer oder wässeriger Lösung sind zur Erzeugung des nascierenden Wasserstoffs geeignet. Die Umsetzung kann auch in heterogener Phase durchgeführt werden, wobei man zweckmäßig eine wässerige und eine Benzol- oder Toluol-Phase verwendet.

Als Reduktionsmittel können ferner besonders vorteilhaft komplexe Metallhydride, wie $LiAlH_4$, $NaBH_4$, Diisobutylaluminiumhydrid oder $NaAl(OCH_2CH_2OCH_3)_2H_2$ sowie Diboran eingesetzt werden, falls erwünscht unter Zusatz von Katalysatoren wie $BF_3$, $AlCl_3$ oder LiBr. Als Lösungsmittel eignen sich hierfür insbesondere Ether wie Diethylether, Di-n-butylether, THF, Dioxan, Diglyme oder 1,2-Dimethoxyethan sowie Kohlenwasserstoffe wie Benzol. Für eine Re-

duktion mit $NaBH_4$ sind in erster Linie Alkohole wie Methanol oder Ethanol, ferner Wasser sowie wässerige Alkohole als Lösungsmittel geeignet. Nach diesen Methoden reduziert man vorzugsweise bei Temperaturen zwischen -80 und +150°, insbesondere zwischen etwa 0 und etwa 100°.

Besonders vorteilhaft lassen sich -CO-Gruppen in Säureamiden (z.B. solchen der Formel V, worin L eine $-(CH_2)_{n-1}-CO-$ oder -CH=CH-CO-Gruppe bedeutet) mit $LiAlH_4$ in THF bei Temperaturen zwischen etwa 0 und 66° zu $CH_2$-Gruppen reduzieren. Dabei können in 1-Stellung des Indolring befindliche Arylsulfonyl-Schutzgruppen gleichzeitig reduktiv abgespalten werden.

Eine Reduktion der Pyridiniumsalze der Formel V

(worin Q 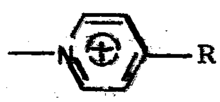 ≙ An$^{\ominus}$ und An vorzugsweise Cl, Br oder $CH_3SO_3$ bedeutet) zu Verbindungen der Formel I gelingt z. B. mit $NaBH_4$ in Waser, Methanol oder Ethanol oder in Gemischen dieser Lösungsmittel, falls erwünscht unter Zusatz einer Base wie NaOH, bei Temperaturen zwischen etwa 0 und 80°.

N-Benzylgruppen können reduktiv mit Natrium in flüssigem Ammoniak abgespalten werden.

Es ist ferner möglich, eine oder mehrere Carbonylgruppen nach der Methode von Wolff-Kishner zu $CH_2$-Gruppen zu reduzieren, z. B. durch Behandlung mit wasserfreiem Hydrazin in absolutem Ethanol unter Druck bei Temperaturen zwischen etwa 150 und 250°. Als Katalysator wird vorteilhaft Natriumalkoholat verwendet. Die Reduktion kann auch nach der Methode von Huang-Minlon variiert werden, indem man mit Hydrazinhydrat in einem hochsiedenden, mit Wasser mischbaren Lösungsmittel, wie Diethylenglykol oder Triethylenglykol, in Gegenwart von Alkali, wie Natriumhydroxid, umsetzt. Das Reaktionsgemisch wird in der Regel etwa 3-4 Stunden gekocht. Anschließend wird das Wasser abdestilliert und das gebildete Hydrazon bei Temperaturen bis zu etwa 200° zersetzt. Die Wolff-Kishner-Reduktion kann auch bei Raumtemperatur in Dimethylsulfoxid mit Hydrazin ausgeführt werden.

Verbindungen, die sonst der Formel I entsprechen, aber an Stelle eines oder mehrerer H-Atome eine oder mehrere solvolysierbare Gruppe(n) enthalten, können zu den Verbindungen der Formel I solvolysiert, insbesondere hydrolysiert werden.

Die Ausgangsstoffe für die Solvolyse sind beispielsweise erhältlich durch Reaktion von IIIa mit Verbindungen, die der Formel II ($X^1$ = X) entsprechen, aber an Stelle eines oder mehrerer H-Atome eine oder mehrere solvolysierbare Gruppe(n) enthalten. So können insbesondere 1-Acylindolderivate (entsprechend der

Formel I, aber in 1-Stellung des Ind-Rests eine Acylgruppe enthaltend, vorzugsweise eine Alkanoyl-, Alkylsulfonyl- oder Arylsulfonylgruppe mit jeweils bis zu 10 C-Atomen, wie Methan-, Benzol- oder p-Toluolsulfonyl) zu den entsprechenden in der 1-Stellung des Indolringes unsubstituierten Indolderivaten hydrolysiert werden, z. B. in saurem, besser in neutralem oder alkalischem Medium bei Temperaturen zwischen 0 und 200°.

Als basische Katalysatoren verwendet man zweckmäßig Natrium-, Kalium- oder Calciumhydroxid, Natrium- oder Kaliumcarbonat, oder Ammoniak. Als Lösungsmittel wählt man vorzugsweise Wasser; niedere Alkohole wie Methanol, Ethanol; Ether wie THF, Dioxan; Sulfone wie Tetramethylensulfon; oder deren Gemische, besonders die Wasser enthaltenden Gemische. Eine Hydrolyse kann auch bereits beim Behandeln mit Wasser allein erfolgen, insbesondere in der Siedehitze.

Man gelangt ferner zu Verbindungen der Formel I, indem man aus Verbindungen der Formel IV unter Ausbildung einer Doppelbindung HE abspaltet. Entsprechend der Definition von E kann es sich z. B. handeln um eine Abspaltung von Halogenwasserstoff, Wasser (Dehydratisierung), einer Carbonsäure oder einer anderen Säure, von Ammoniak oder von HCN. Die Ausgangsstoffe der Formel IV sind z.B. erhältlich durch Umsetzung von II ($X^1$ = X) mit einer Verbindung der Formel VII

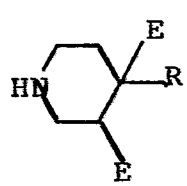

VII

worin E und R die angegebenen Bedeutungen haben.

Falls einer der Reste E = Hal ist, kann dieser Substituent unter basischen Reaktionsbedingungen leicht eliminiert werden. Als Basen können verwendet werden: Alkalimetallhydroxide, Alkalimetallcarbonate, Alkoholate, wie z. B. Kalium-tert.-butylat, Amine, wie z. B. Dimethylanilin, Pyridin, Collidin oder Chinolin; als Lösungsmittel benutzt man z. B. Benzol, Toluol, Cyclohexan, THF oder tert.-Butanol. Die als Basen verwendeten Amine können auch im Überschuß als Lösungsmittel eingesetzt werden. Bedeutet der eine der Reste E eine OH-Gruppe, so benutzt man als wasserabspaltende Mittel vorzugsweise Säuren wie Essigsäure, Salzsäure oder Gemische beider. Der Zusatz eines Lösungsmittels (z. B. Wasser oder Ethanol) kann von Vorteil sein. Die Eliminierung von Acyl-, Alkylsulfonyl- sowie Alkoxysulfonyl-oxy- oder Amino-Resten kann unter ähnlichen Bedingungen durchgeführt werden. Eine Eliminierung von Sulfonsäure-Resten, z. B. die der Mesylate oder Tosylate, erfolgt schonend durch Kochen in DMF oder Dimethylsulfoxid mit Alkalimetallcarbonaten, z. B. $Li_2CO_3$, oder mit Kaliumacetat. Ammoniak kann bereits durch Erhitzen der Salze der entsprechenden Aminoverbindungen (insbesondere der 4-Aminoderivate) abgespalten werden. In ähnlicher Weise kann HCN aus Verbindungen der Formel IV (eine Gruppe E = CN) durch Erhitzen abgespalten werden. Die Eliminierung von HE aus IV erfolgt allgemein bei Temperaturen zwischen 0 und etwa 250°, vorzugsweise zwischen 50 und 200°.

Weiterhin kann man gegebenenfalls eine Verbindung der Formel I nach an sich bekannten Methoden in eine andere Verbindung der Formel I umwandeln.

So können Ether (O-Alkylderivate) gespalten werden, wobei die entsprechenden Hydroxyderivate entstehen. Z.B. kann man die Ether spalten durch Behandeln mit Dimethylsulfid-Bortribromid-Komplex, z.B. in Toluol, 1,2-Dichlorethan, THF oder Dimethylsulfoxid, oder durch Verschmelzen mit Pyridin- oder Anilin-hydrohalogeniden, vorzugsweise Pyridinhydrochlorid, bei etwa 150-250°.

Eine erhaltene Base der Formel I kann mit einer Säure in das zugehörige Säureadditionssalz übergeführt werden. Für diese Umsetzung eignen sich bevorzugt Säuren, die physiologisch unbedenkliche Salze liefern. So können anorganische Säuren verwendet werden. z.B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Säureadditionssalze, die nicht physiologisch unbedenklich sind (z.B. Pikrate), können sich zur Isolierung und Aufreinigung von Basen der Formel I eignen.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Wege. Hierbei können sie zusammen mit mindestens einem Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze. Diese Zubereitungen können als Arzneimittel in der Human- oder Veterinärmedizin eingesetzt werden. Als Trägersubstanzen kommen organische oder anorganische Stoffe in Frage, die sich für die enterale (z. B. orale), parenterale oder topische Applikation eignen und mit den neuen Verbindungen nicht reagieren, beispielsweise Wasser, pflanzliche Öle, Benzylalkohole, Polyethylenglykole, Gelatine, Kohlehydrate wie Lactose oder Stärke, Magnesiumstearat, Talk, Vaseline. Zur enteralen Applikation dienen insbesondere Tabletten, Dragees, Kapseln, Sirupe, Säfte, Tropfen oder Suppositorien, zur parenteralen Applikation Lösungen, vorzugsweise ölige oder wässerige Lösungen, fernerSuspensionen, Emulsionen oder Implantate, für

die topische Anwendung Salben, Cremes oder Puder. Die neuen Verbindungen können auch lyophilisiert und die erhaltenen Lyophilisate z.B. zur Herstellung von Injektionspräparaten verwendet werden.

Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Gegenstand der Erfindung ist ferner die Verwendung der Verbindungen der Formel I und ihrer physiologisch unbedenklichen Salze bei der therapeutischen Behandlung des menschlichen oder tierischen Körpers und bei der Bekämpfung von Krankheiten, insbesondere von Schizophrenie und psychoreaktiven Störungen und Psychopathien, von Depressionen, von schweren chronischen Schmerzen und von Krankheiten, die mit erhöhtem Blutdruck einhergehen.
Weiterhin können die Verbindungen bei der Behandlung extrapyramidaler Störungen Anwendung finden.

Dabei werden die erfindungsgemäßen Substanzen in der Regel in Analogie zu bekannten, im Handel befindlichen Präparaten (Thioridazin, Dihydroergocristin) verabreicht, vorzugsweise in Dosierungen zwischen etwa 0,2 und 500 mg, insbesondere zwischen 0,2 und 50 mg pro Dosierungseinheit. Die tägliche Dosierung liegt vorzugsweise zwischen etwa 0,003 und 10 mg/kg Körpergewicht.

Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

In den nachstehenden Beispielen bedeutet "übliche Aufarbeitung": Man gibt, falls erforderlich, Wasser hinzu, extrahiert mit Dichlormethan, trennt ab, trocknet die organische Phase über Natriumsulfat, filtriert, dampft ein und reinigt durch Chromatographie an Kieselgel und/oder durch Kristallisation. Temperaturen sind in °C angegeben. Rf-Werte wurden dünnschichtchromatographisch an Kieselgel ermittelt.

Beispiel 1

Man rührt eine Lösung von 26,7 g 4-(4-Methylsulfonyloxybutyl)-indol /erhältlich durch Reaktion von 4-Formylindol mit Malonsäure sowie anschließende Decarboxylierung zu 3-(Indol-4-yl)-acrylsäure, Reduktion mit $LiAlH_4$ in THF zu 3-(Indol-4-yl)-propan-1-ol (Rf 0,5 in $CH_2Cl_2/CH_3OH$ 95 : 5), Mesylierung zum Methansulfonat (Rf 0,8 in $CH_2Cl_2/CH_3OH$ 98 : 2), Umsetzung mit NaCN in Dimethylsulfoxid zu 4-(3-Cyanpropyl)-indol (Rf 0,75 in $CH_2Cl_2/CH_3OH$ 98 : 2), Hydrolyse mit NaOH in Diethylenglykolmonoethylether zu 4-(3-Carboxypropyl)-indol (Rf 0,45 in $CH_2Cl_2/CH_3OH$), $LiAlH_4$-Reduktion zu 4-(4-Hydroxybutyl)-indol und Mesylierung/ und 16 g 4-Phenyl-1,2,3,6-tetrahydropyridin in 100 ml Acetonitril 12 Std. bei 20°, arbeitet wie üblich auf und erhält 4-/4-(4-Phenyl-1,2,3,6-tetrahydropyridyl)-butyl/-indol, F. 133 - 135°.

Analog erhält man aus den entsprechenden Methansulfonaten, Chloriden oder Bromiden:

4-/2-(4-Phenyl-1,2,3,6-tetrahydropyridyl)-ethyl/-indol, F. 128 - 130°

4-/2-(4-Phenyl-1,2,3,6-tetrahydropyridyl)-ethyl/-2-methylindol

4-/2-(4-Phenyl-1,2,3,6-tetrahydropyridyl)-ethyl/-3-methylindol

4-/2-(4-Phenyl-1,2,3,6-tetrahydropyridyl)-ethyl/-5-methylindol

4-/2-(4-Phenyl-1,2,3,6-tetrahydropyridyl)-ethyl/-6-methylindol

4-/2-(4-Phenyl-1,2,3,6-tetrahydropyridyl)-ethyl/-7-methylindol

4-/2-(4-Phenyl-1,2,3,6-tetrahydropyridyl)-ethyl/-5-methoxyindol

4-/2-(4-Phenyl-1,2,3,6-tetrahydropyridyl)-ethyl/-6-methoxyindol

4-[2-(4-Phenyl-1,2,3,6-tetrahydropyridyl)-ethyl]-7-methoxyindol

4-[2-(4-Phenyl-1,2,3,6-tetrahydropyridyl)-ethyl]-2-hydroxyindol

4-[2-(4-Phenyl-1,2,3,6-tetrahydropyridyl)-ethyl]-5-hydroxindol

4-[2-(4-Phenyl-1,2,3,6-tetrahydropyridyl)-ethyl]-6-hydroxyindol

4-[2-(4-Phenyl-1,2,3,6-tetrahydropyridyl)-ethyl]-7-hydroxyindol

4-[2-(4-Phenyl-1,2,3,6-tetrahydropyridyl)-ethyl]-5-fluorindol

4-[2-(4-Phenyl-1,2,3,6-tetrahydropyridyl)-ethyl]-6-fluorindol

4-[2-(4-Phenyl-1,2,3,6-tetrahydropyridyl)-ethyl]-7-fluorindol

4-[2-(4-Phenyl-1,2,3,6-tetrahydropyridyl)-ethyl]-5-chlorindol

4-[2-(4-Phenyl-1,2,3,6-tetrahydropyridyl)-ethyl]-6-chlorindol

4-[2-(4-Phenyl-1,2,3,6-tetrahydropyridyl)-ethyl]-7-chlorindol

4-[2-(4-Phenyl-1,2,3,6-tetrahydropyridyl)-ethyl]-5,6-dimethoxyindol

2-Hydroxy-4-[2-(4-phenyl-1,2,3,6-tetrahydropyridyl)-ethyl]-5-methoxyindol

4-[2-(4-o-Tolyl-1,2,3,6-tetrahydropyridyl)-ethyl]-indol

4-[2-(4-m-Tolyl-1,2,3,6-tetrahydropyridyl)-ethyl]-indol

4-[2-(4-p-Tolyl-1,2,3,6-tetrahydropyridyl)-ethyl]-indol

4-[2-(4-o-Fluorphenyl-1,2,3,6-tetrahydropyridyl)-ethyl]-indol

4-[2-(4-p-Fluorphenyl-1,2,3,6-tetrahydropyridyl)-ethyl]-indol

4-/2-(4-o-Chlorphenyl-1,2,3,6-tetrahydropyridyl)-ethyl7-indol

4-/2-(4-p-Chlorphenyl-1,2,3,6-tetrahydropyridyl)-ethyl7-indol

4-/2-(4-m-Trifluormethylphenyl-1,2,3,6-tetrahydropyridyl)-ethyl7-indol

4-/2-(4-(2,4-Dichlorphenyl)-1,2,3,6-tetrahydropyridyl)-ethyl7-indol

4-/2-(4-(2,6-Dichlorphenyl)-1,2,3,6-tetrahydropyridyl)-ethyl7-indol

4-/2-(4-(2-Thienyl)-1,2,3,6-tetrahydropyridyl)-ethyl7-indol

4-/3-(4-Phenyl-1,2,3,6-tetrahydropyridyl)-propyl7-indol , F. 128-130°

4-/3-(4-Phenyl-1,2,3,6-tetrahydropyridyl)-propyl7-2-methylindol

4-/3-(4-Phenyl-1,2,3,6-tetrahydropyridyl)-propyl7-3-methylindol

4-/3-(4-Phenyl-1,2,3,6-tetrahydropyridyl)-propyl7-5-methylindol

4-/3-(4-Phenyl-1,2,3,6-tetrahydropyridyl)-propyl7-6-methylindol

4-/3-(4-Phenyl-1,2,3,6-tetrahydropyridyl)-propyl7-7-methylindol

4-/3-(4-Phenyl-1,2,3,6-tetrahydropyridyl)-propyl7-5-methoxyindol

4-/3-(4-Phenyl-1,2,3,6-tetrahydropyridyl)-propyl7-6-methoxyindol

4-/3-(4-Phenyl-1,2,3,6-tetrahydropyridyl)-propyl7-7-methoxyindol

4-/3-(4-Phenyl-1,2,3,6-tetrahydropyridyl)-propyl7-2-hydroxyindol

4-/3-(4-Phenyl-1,2,3,6-tetrahydropyridyl)-propyl7-5-hydroxyindol

4-/3-(4-Phenyl-1,2,3,6-tetrahydropyridyl)-propyl7-6-hydroxyindol

4-[3-(4-Phenyl-1,2,3,6-tetrahydropyridyl)-propyl]-7-hydroxyindol

4-[3-(4-Phenyl-1,2,3,6-tetrahydropyridyl)-propyl]-5-fluorindol

4-[3-(4-Phenyl-1,2,3,6-tetrahydropyridyl)-propyl]-6-fluorindol

4-[3-(4-Phenyl-1,2,3,6-tetrahydropyridyl)-propyl]-7-fluorindol

4-[3-(4-Phenyl-1,2,3,6-tetrahydropyridyl)-propyl]-5-chlorindol

4-[3-(4-Phenyl-1,2,3,6-tetrahydropyridyl)-propyl]-6-chlorindol

4-[3-(4-Phenyl-1,2,3,6-tetrahydropyridyl)-propyl]-7-chlorindol

4-[3-(4-Phenyl-1,2,3,6-tetrahydropyridyl)-propyl]-5,6-dimethoxyindol

2-Hydroxy-4-[3-(4-phenyl-1,2,3,6-tetrahydropyridyl)-propyl]-5-methoxyindol

4-[3-(4-o-Tolyl-1,2,3,6-tetrahydropyridyl)-propyl]-indol

4-[3-(4-m-Tolyl-1,2,3,6-tetrahydropyridyl)-propyl]-indol

4-[3-(4-p-Tolyl-1,2,3,6-tetrahydropyridyl)-propyl]-indol

4-[3-(4-o-Fluorphenyl-1,2,3,6-tetrahydropyridyl)-propyl]-indol

4-[3-(4-p-Fluorphenyl-1,2,3,6-tetrahydropyridyl)-propyl]-indol, F. 121-122°

4-[3-(4-o-Chlorphenyl-1,2,3,6-tetrahydropyridyl)-propyl]-indol

4-[3-(4-p-Chlorphenyl-1,2,3,6-tetrahydropyridyl)-propyl]-indol

4-[3-(4-m-Trifluormethylphenyl-1,2,3,6-tetrahydropyridyl)-propyl]-indol

4-/3-(4-(2,4-Dichlorphenyl)-1,2,3,6-tetrahydropyridyl)-propyl/-indol

4-/3-(4-(2,6-Dichlorphenyl)-1,2,3,6-tetrahydropyridyl)-propyl/-indol

4-/3-(4-(2-Thienyl)-1,2,3,6-tetrahydropyridyl)-propyl/-indol

4-/4-(4-Phenyl-1,2,3,6-tetrahydropyridyl)-butyl/-2-methylindol

4-/4-(4-Phenyl-1,2,3,6-tetrahydropyridyl)-butyl/-3-methylindol

4-/4-(4-Phenyl-1,2,3,6-tetrahydropyridyl)-butyl/-5-methylindol

4-/4-(4-Phenyl-1,2,3,6-tetrahydropyridyl)-butyl/-6-methylindol

4-/4-(4-Phenyl-1,2,3,6-tetrahydropyridyl)-butyl/-7-methylindol

4-/4-(4-Phenyl-1,2,3,6-tetrahydropyridyl)-butyl/-5-methoxyindol

4-/4-(4-Phenyl-1,2,3,6-tetrahydropyridyl)-butyl/-6-methoxyindol

4-/4-(4-Phenyl-1,2,3,6-tetrahydropyridyl)-butyl/-7-methoxyindol

4-/4-(4-Phenyl-1,2,3,6-tetrahydropyridyl)-butyl/-2-hydroxyindol

4-/4-(4-Phenyl-1,2,3,6-tetrahydropyridyl)-butyl/-5-hydroxyindol

4-/4-(4-Phenyl-1,2,3,6-tetrahydropyridyl)-butyl/-6-hydroxyindol

4-/4-(4-Phenyl-1,2,3,6-tetrahydropyridyl)-butyl/-7-hydroxyindol

4-/4-(4-Phenyl-1,2,3,6-tetrahydropyridyl)-butyl/-5-fluorindol

4-/4-(4-Phenyl-1,2,3,6-tetrahydropyridyl)-butyl/-6-fluorindol

4-/4-(4-Phenyl-1,2,3,6-tetrahydropyridyl)-butyl/-7-fluorindol

4-/4-(4-Phenyl-1,2,3,6-tetrahydropyridyl)-butyl7-5-chlorindol

4-/4-(4-Phenyl-1,2,3,6-tetrahydropyridyl)-butyl7-6-chlorindol

4-/4-(4-Phenyl-1,2,3,6-tetrahydropyridyl)-butyl7-7-chlorindol

4-/4-(4-Phenyl-1,2,3,6-tetrahydropyridyl)-butyl7-5,6-dimethoxyindol

2-Hydroxy-4-/4-(4-phenyl-1,2,3,6-tetrahydropyridyl)-butyl7-5-methoxyindol

4-/4-(4-o-Tolyl-1,2,3,6-tetrahydropyridyl)-butyl7-indol

4-/4-(4-m-Tolyl-1,2,3,6-tetrahydropyridyl)-butyl7-indol

4-/4-(4-p-Tolyl-1,2,3,6-tetrahydropyridyl)-butyl7-indol

4-/4-(4-o-Fluorphenyl-1,2,3,6-tetrahydropyridyl)-butyl7-indol

4-/4-(4-p-Fluorphenyl-1,2,3,6-tetrahydropyridyl)-butyl7-indol

4-/4-(4-o-Chlorphenyl-1,2,3,6-tetrahydropyridyl)-butyl7-indol

4-/4-(4-p-Chlorphenyl-1,2,3,6-tetrahydropyridyl)-butyl7-indol

4-/4-(4-m-Trifluormethylphenyl-1,2,3,6-tetrahydropyridyl)-butyl7-indol

4-/4-(4-(2,4-Dichlorphenyl)-1,2,3,6-tetrahydropyridyl)-butyl7-indol

4-/4-(4-(2,6-Dichlorphenyl),1,2,3,6-tetrahydropyridyl)-butyl7-indol

4-/4-(4-(2-Thienyl)-1,2,3,6-tetrahydropyridyl)-butyl7-indol.

4-/3-(4-Phenyl-1,2,3,6-tetrahydropyridyl)-1-propenyl7-indol, F. 183-185°

Beispiel 2

Ein Gemisch von 1,6 g 4-(2-Aminoethyl)-indol (erhältlich durch $LiAlH_4$-Reduktion von 4-Cyanmethylindol) und 2,15 g 1,5-Dichlor-3-phenyl-2-penten in 40 ml Aceton und 40 ml Wasser wird 24 Stunden gekocht und wie üblich aufgearbeitet.
Man erhält 4-[2-(4-Phenyl-1,2,3,6-tetrahydropyridyl)-ethyl]-indol, F. 128-130°.

Beispiel 3

Eine Lösung von 32,8 g 4-[3-(4-Phenyl-1,2,3,6-tetrahydropyridyl)-3-oxo-1-propenyl]-indol (F. 203-205°; erhältlich durch Reaktion von 3-(Indol-4-yl)-acrylsäure mit 4-Phenyl-1,2,3,6-tetrahydropyridin in Gegenwart von Carbonyldiimidazol in THF] in 200 ml THF wird zu einer Suspension von 6 g $LiAlH_4$ in 100 ml THF unter Rühren hinzugetropft. Man rührt noch 1 Stunde bei 20°, arbeitet wie üblich auf und erhält 4-[3-(4-Phenyl-1,2,3,6-tetrahydropyridyl)-1-propenyl]-indol, F. 183-185° (aus Ethanol).

Analog erhält man aus den entsprechenden Säureamiden:

4-[3-(4-Phenyl-1,2,3,6-tetrahydropyridyl)-1-propenyl]-2-methylindol
4-[3-(4-Phenyl-1,2,3,6-tetrahydropyridyl)-1-propenyl]-3-methylindol
4-[3-(4-Phenyl-1,2,3,6-tetrahydropyridyl)-1-propenyl]-5-methylindol
4-[3-(4-Phenyl-1,2,3,6-tetrahydropyridyl)-1-propenyl]-6-methylindol
4-[3-(4-Phenyl-1,2,3,6-tetrahydropyridyl)-1-propenyl]-7-methylindol

4-[3-(4-Phenyl-1,2,3,6-tetrahydropyridyl)-1-propenyl]-2-ethylindol

4-[3-(4-Phenyl-1,2,3,6-tetrahydropyridyl)-1-propenyl]-2-butylindol

4-[3-(4-Phenyl-1,2,3,6-tetrahydropyridyl)-1-propenyl]-5-methoxyindol, F. 184-185°

4-[3-(4-Phenyl-1,2,3,6-tetrahydropyridyl)-1-propenyl]-6-methoxyindol

4-[3-(4-Phenyl-1,2,3,6-tetrahydropyridyl)-1-propenyl]-7-methoxyindol

4-[3-(4-Phenyl-1,2,3,6-tetrahydropyridyl)-1-propenyl]-5-ethoxyindol

4-[3-(4-Phenyl-1,2,3,6-tetrahydropyridyl)-1-propenyl]-5-butoxyindol

4-[3-(4-Phenyl-1,2,3,6-tetrahydropyridyl)-1-propenyl]-5-methylthioindol

4-[3-(4-Phenyl-1,2,3,6-tetrahydropyridyl)-1-propenyl]-6-methylthioindol

4-[3-(4-Phenyl-1,2,3,6-tetrahydropyridyl)-1-propenyl]-7-methylthioindol

4-[3-(4-Phenyl-1,2,3,6-tetrahydropyridyl)-1-propenyl]-5-ethylthioindol

4-[3-(4-Phenyl-1,2,3,6-tetrahydropyridyl)-1-propenyl]-5-butylthioindol

4-[3-(4-Phenyl-1,2,3,6-tetrahydropyridyl)-1-propenyl]-2-hydroxyindol

4-[3-(4-Phenyl-1,2,3,6-tetrahydropyridyl)-1-propenyl]-3-hydroxyindol

4-[3-(4-Phenyl-1,2,3,6-tetrahydropyridyl)-1-propenyl]-5-hydroxyindol

4-[3-(4-Phenyl-1,2,3,6-tetrahydropyridyl)-1-propenyl]-6-hydroxyindol

4-[3-(4-Phenyl-1,2,3,6-tetrahydropyridyl)-1-propenyl]-7-hydroxyindol

4-/3-(4-Phenyl-1,2,3,6-tetrahydropyridyl)-1-propenyl7-5-fluorindol

4-/3-(4-Phenyl-1,2,3,6-tetrahydropyridyl)-1-propenyl7-6-fluorindol

4-/3-(4-Phenyl-1,2,3,6-tetrahydropyridyl)-1-propenyl7-7-fluorindol

4-/3-(4-Phenyl-1,2,3,6-tetrahydropyridyl)-1-propenyl7-5-chlorindol

4-/3-(4-Phenyl-1,2,3,6-tetrahydropyridyl)-1-propenyl7-6-chlorindol

4-/3-(4-Phenyl-1,2,3,6-tetrahydropyridyl)-1-propenyl7-7-chlorindol

4-/3-(4-Phenyl-1,2,3,6-tetrahydropyridyl)-1-propenyl7-5-bromindol

4-/3-(4-Phenyl-1,2,3,6-tetrahydropyridyl)-1-propenyl7-6-bromindol

4-/3-(4-Phenyl-1,2,3,6-tetrahydropyridyl)-1-propenyl7-7-bromindol

4-/3-(4-Phenyl-1,2,3,6-tetrahydropyridyl)-1-propenyl7-5,6-dimethoxyindol

2-Hydroxy-4-/3-(4-phenyl-1,2,3,6-tetrahydropyridyl)-1-propenyl7-5-methoxyindol

4-/3-(4-o-Tolyl-1,2,3,6-tetrahydropyridyl)-1-propenyl7-indol

4-/3-(4-m-Tolyl-1,2,3,6-tetrahydropyridyl)-1-propenyl7-indol

4-/3-(4-p-Tolyl-1,2,3,6-tetrahydropyridyl)-1-propenyl7-indol

4-/3-(4-p-Isobutylphenyl-1,2,3,6-tetrahydropyridyl)-1-propenyl7-indol

4-/3-(4-o-Fluorphenyl-1,2,3,6-tetrahydropyridyl)-1-propenyl7-indol

4-/3-(4-m-Fluorphenyl-1,2,3,6-tetrahydropyridyl)-1-propenyl7-indol

4-/3-(4-p-Fluorphenyl-1,2,3,6-tetrahydropyridyl)-1-propenyl7-indol

4-/3-(4-o-Chlorphenyl-1,2,3,6-tetrahydropyridyl)-1-propenyl7-indol
4-/3-(4-m-Chlorphenyl-1,2,3,6-tetrahydropyridyl)-1-propenyl7-indol
4-/3-(4-p-Chlorphenyl-1,2,3,6-tetrahydropyridyl)-1-propenyl7-indol
4-/3-(4-o-Bromphenyl-1,2,3,6-tetrahydropyridyl)-1-propenyl7-indol
4-/3-(4-m-Bromphenyl-1,2,3,6-tetrahydropyridyl)-1-propenyl7-indol
4-/3-(4-p-Bromphenyl-1,2,3,6-tetrahydropyridyl)-1-propenyl7-indol
4-/3-(4-o-Trifluormethylphenyl-1,2,3,6-tetrahydropyridyl)-1-propenyl7-indol
4-/3-(4-m-Trifluormethylphenyl-1,2,3,6-tetrahydropyridyl)-1-propenyl7-indol, Rf 0,42 (Toluol/Triethylamin 8:2)
4-/3-(4-p-Trifluormethylphenyl-1,2,3,6-tetrahydropyridyl)-1-propenyl7-indol
4-/3-(4-(2,4-Dichlorphenyl)-1,2,3,6-tetrahydropyridyl)-1-propenyl7-indol
4-/3-(4-(2,6-Dichlorphenyl)-1,2,3,6-tetrahydropyridyl)-1-propenyl7-indol
4-/3-(4-(2-Thienyl)-1,2,3,6-tetrahydropyridyl)-1-propenyl7-indol, F. 156-158°
4-/3-(4-(3-Thienyl)-1,2,3,6-tetrahydropyridyl)-1-propenyl7-indol.

Analog sind die in Beispiel 1 genannten Verbindungen erhältlich.

Beispiel 4

Zu einer Lösung von 4,07 g 1-/4-(4-Indolyl)-butyl7-4-phenyl-pyridiniumbromid /erhältlich aus 4-(4-Brombutyl)-indol und 4-Phenylpyridin7 in 50 ml 1 n NaOH

gibt man unter Rühren 1 g $NaBH_4$ in 20 ml Wasser und rührt danach noch 3 Stunden bei 60°. Nach üblicher Aufarbeitung erhält man 4-/4-Phenyl-1,2,3,6-tetrahydropyridyl)-butyl7-indol, F. 133-135°.

Analog erhält man durch Reduktion der entsprechenden 4-Phenylpyridiniumbromide:

1-Methyl-4-/4-(4-phenyl-1,2,3,6-tetrahydropyridyl)-butyl7-5-methoxyindol
1-Methyl-4-/4-(4-phenyl-1,2,3,6-tetrahydropyridyl)-butyl7-5,6-dimethoxyindol
4-/2-(4-Phenyl-1,2,3,6-tetrahydropyridyl)-1-methyl-ethyl7-indol.

Beispiel 5

Man kocht 10 g 1-Benzolsulfonyl-4-/3-(4-phenyl-1,2,3,6-tetrahydropyridyl)-1-propenyl7-indol /erhältlich aus 1-Benzolsulfonyl-1-(3-chlor-1-propenyl)-indol und 4-Phenyl-1,2,3,6-tetrahydropyridin7 mit 2 g KOH in 15 ml Wasser und 30 ml Ethanol 16 Stunden, konzentriert das Gemisch, arbeitet wie üblich auf und erhält 4-/3-(4-Phenyl-1,2,3,6-tetrahydropyridyl)-1-propenyl7-indol, F. 183-185°.

Beispiel 6

Man erhitzt 3,62 g 1-Methyl-4-/4-(4-hydroxy-4-phenyl-1-piperidyl)-butyl7-indol /erhältlich durch Reaktion von 1-Methyl-4-(4-brombutyl)-indol mit 4-Piperidon, anschließende Umsetzung mit $C_6H_5Li$ und Hydrolyse7 mit 40 ml 1 n Salzsäure 2 Stunden auf 50°, arbeitet wie üblich auf und erhält 1-Methyl-/4-(4-phenyl-1,2,3,6-tetrahydropyridyl)-butyl7-indol.

Beispiel 7

Ein Gemisch von 10 g 4-/3-(4-Phenyl-1,2,3,6-tetrahydropyridyl)-1-propenyl7-5-methoxyindol und 10 g Pyridinhydrochlorid wird 3 Stunden bei 160° gerührt. Nach üblicher Aufarbeitung erhält man 4-/3-(4-Phenyl-1,2,3,6-tetrahydropyridyl)-1-propenyl7-5-hydroxyindol.

Beispiel 8

Man tropft eine Suspension von 3,44 g 4-/3-(4-Phenyl-1,2,3,6-tetrahydropyridyl)-1-propenyl7-5-methoxyindol in 50 ml 1,2-Dichlorethan zu einer siedenden Lösung von 15,6 g Dimethylsulfid-Bortribromid-Komplex in 50 ml 1,2-Dichlorethan, kocht noch 30 Minuten, arbeitet wie üblich auf und erhält 4-/3-(4-Phenyl-1,2,3,6-tetrahydropyridyl)-1-propenyl7-5-hydroxyindol.

Die nachstehenden Beispiele betreffen pharmazeutische Zubereitungen, die Amine der Formel I oder ihre Säureadditionssalze enthalten:

Beispiel A: Tabletten

Ein Gemisch von 1 kg 4-/3-(4-Phenyl-1,2,3,6-tetrahydropyridyl)-1-propenyl7-indol, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

Beispiel B: Dragees

Analog Beispiel A werden Tabletten gepreßt, die anschließend in üblicher Weise mit einem Überzug aus Saccharose, Kartoffelstärke, Talk, Tragant und Farbstoff überzogen werden.

Beispiel C: Kapseln

2 kg 4-/3-(4-Phenyl-1,2,3,6-tetrahydropyridyl)-1-propenyl7-indol werden in üblicher Weise in Hartgelatinekapseln gefüllt, so daß jede Kapsel 20 mg des Wirkstoffs enthält.

Beispiel D: Ampullen

Eine Lösung von 1 kg 4-/3-(4-Phenyl-1,2,3,6-tetrahydropyridyl)-1-propenyl7-indol-methansulfonat in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

Analog sind Tabletten, Dragees, Kapseln und Ampullen erhältlich, die einen oder mehrere der übrigen Wirkstoffe der Formel I und/oder ihre physiologisch unbedenklichen Säureadditionssalze enthalten.

Merck Patent Gesellschaft
mit beschränkter Haftung
D a r m s t a d t

Patentansprüche:

1. Indolderivate der Formel I

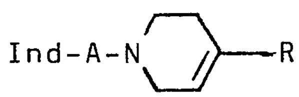

I

worin

Ind einen unsubstituierten oder ein- bis dreifach durch Alkyl, O-Alkyl, S-Alkyl, OH, F, Cl und/oder Br substituierten Indol-4-yl-rest,

A $-C_nH_{2n}-$ oder $-CH=CH-CH_2-$,

R einen unsubstituierten oder ein- oder zweifach durch Alkyl, F, Cl, Br und/oder $CF_3$ substituierten Phenyl- oder 2- oder 3-Thienylrest und

n 2,3 oder 4

bedeuten und die Alkylgruppen jeweils 1 - 4 C-Atome besitzen,
sowie deren Salze.

2. 4-/3-(4-Phenyl-1,2,3,6-tetrahydropyridyl)-1-propenyl7-indol.

3. Verfahren zur Herstellung von Indolderivaten der Formel I nach Anspruch 1 sowie von deren Salzen, dadurch gekennzeichnet, daß man eine Verbindung der Formel II

$$Ind\text{-}A\text{-}X^1 \qquad II$$

worin

$X^1$ X oder $NH_2$ und

X Cl, Br, J, OH oder eine reaktionsfähig funktionell abgewandelte OH-Gruppe bedeuten und

Ind und A die angegebenen Bedeutungen haben,

mit einer Verbindung der Formel III

$$X^2\text{-}CH_2CH_2\text{-}CR{=}CH\text{-}CH_2\text{-}X^3 \qquad III$$

worin

$X^2$ und $X^3$ gleich oder verschieden sind und, falls $X^1 = NH_2$ ist, jeweils X, andernfalls zusammen NH bedeuten und

R die angegebene Bedeutung hat,

umsetzt

oder daß man eine sonst der Formel I entsprechende Verbindung, die jedoch an Stelle eines oder mehrerer Wasserstoffatome eine oder mehrere reduzierbare Gruppen und/oder eine oder mehrere zusätzliche C-C- und/oder C-N-Bindungen enthält, mit einem reduzierenden Mittel behandelt

oder daß man eine sonst der Formel I entsprechende Verbindung, die jedoch an Stelle eines oder mehrerer Wasserstoffatome eine oder mehrere solvolytisch abspaltbare Gruppen enthält, mit einem solvolysierenden Mittel behandelt

oder daß man eine Verbindung der Formel IV

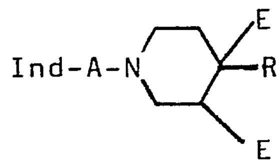

IV

worin

der eine Rest E X, CN oder $NH_2$,

der andere Rest E H bedeutet und

Ind, A, R und X die angegebenen Bedeutungen haben

mit einem HE-abspaltenden Mittel behandelt

und/oder daß man gegebenenfalls in einer Verbindung der Formel I eine O-Alkylgruppe unter Bildung einer OH-Gruppe spaltet

und/oder daß man eine Base der Formel I durch Behandeln mit einer Säure in eines ihrer Salze umwandelt.

4. Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfsstoff und gegebenenfalls in Kombination mit einem oder mehreren weiteren Wirkstoff(en) in eine geeignete Dosierungsform bringt.

5. Pharmazeutische Zubereitung, gekennzeichnet durch einen Gehalt an mindestens einer Verbindung der Formel I und/oder einem ihrer physiologisch unbedenklichen Salze.

6. Verbindungen der Formel I nach Patentanspruch 1 zur Bekämpfung von Krankheiten.

7. Verwendung von Verbindungen der Formel I bei der Bekämpfung von Krankheiten.